(19) Europäisches Patentamt
European Patent Office
Office européen des brevets

(11) Veröffentlichungsnummer : **0 438 376 B1**

# (12) EUROPÄISCHE PATENTSCHRIFT

(45) Veröffentlichungstag der Patentschrift : **26.01.94 Patentblatt 94/04**

(51) Int. Cl.$^5$ : **C07C 323/22**, C07C 317/24, C09K 9/02, G03C 1/73

(21) Anmeldenummer : **91810009.0**

(22) Anmeldetag : **09.01.91**

## (54) Photochrome Naphthacenchinone, Verfahren zu deren Herstellung und deren Verwendung.

(30) Priorität : **18.01.90 CH 161/90**

(43) Veröffentlichungstag der Anmeldung : **24.07.91 Patentblatt 91/30**

(45) Bekanntmachung des Hinweises auf die Patenterteilung : **26.01.94 Patentblatt 94/04**

(84) Benannte Vertragsstaaten : **CH DE FR GB IT LI NL**

(56) Entgegenhaltungen :
**EP-A- 0 344 110**
**GB-A- 2 093 475**

(73) Patentinhaber : **CIBA-GEIGY AG**
**Klybeckstrasse 141**
**CH-4002 Basel (CH)**

(72) Erfinder : **Fischer, Walter, Dr.**
**Vogesenstrasse 77**
**CH-4153 Reinach (CH)**
Erfinder : **Fischer, Evelyn, Dr.**
**Schutzackerstrasse 64**
**W-7858 Weil am Rhein (DE)**
Erfinder : **Minder, Ernst**
**Bernhardsmattweg 1**
**CH-4450 Sissach (CH)**
Erfinder : **Hofmann, Manfred, Dr.**
**Route Bel-Air 38**
**CH-1723 Marly (CH)**
Erfinder : **Finter, Jürgen, Dr.**
**Zasiusstrasse 100**
**W-7800 Freiburg (DE)**
Erfinder : **Spahni, Heinz**
**Eggstrasse 23**
**CH-4402 Frenkendorf (CH)**

**EP 0 438 376 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft in 5,12- bzw. 6,11-Stellung mit Aryloxygruppen und in den 2-, 3-, 8- und/oder 9-Stellungen mit mindestens einem organischem Thio-, Sulfoxyl- oder Sulfonylrest substituierte Naphthacen-6,11- bzw. Naphthacen-5,12-dione, entsprechende 5,12- bzw. 6,11-Dichlornaphthacendione mit organischen Thioresten, ein Verfahren zur Herstellung der mit Aryloxygruppen substituierten Naphthacendione und deren Verwendung als photochrome Systeme zur Kontrastbildung oder Lichtabsorption.

Substituierte Naphthacen-5,12-dione sind in EP-A-344 110 beschrieben.

Yu. E. Gerasimenko et al. beschreiben im Zhurnal Organicheskoi Khimii, Vol. 7, No. 11, S. 2413-2415 (1971) 6-Phenoxy-naphthacen-5,12-dion als reversible photochrome Verbindung, die bei Bestrahlung das orange 5-Phenoxynaphthacen-6,12-dion (Anachinon) bildet. Yu. E. Gerasimenko et al. beschreiben im Zhurnal Organicheskoi Khimii, Vol. 16, No. 9, S. 1938-1945 (1980) 6,11-Diphenoxy-naphthacen-5,12-dion, dessen Photoisomerisation zur Synthese von 6-Aminoderivaten des 12-Phenoxy-naphthacen-5,11-dions verwendet wird.

Ein Gegenstand der Erfindung sind Verbindungen der Formel I oder Mischungen solcher Verbindungen,

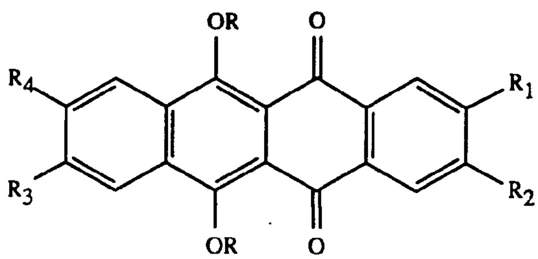

(I),

worin

R unsubstituiertes oder mit $C_1$-$C_{12}$-Alkyl, $C_1$-$C_{12}$-Alkoxy, $C_1$-$C_{12}$-Alkylthio, Phenyl, Benzyl, -CN, $-CF_3$, Halogen oder $-COOR_5$ substituiertes $C_6$-$C_{14}$-Aryl bedeutet und $R_5$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht, und

mindestens einer der Reste $R_1$ bis $R_4$ einen organischen Thio-, Sulfoxyl- oder Sulfonylrest darstellt, und die anderen Reste von $R_1$ bis $R_4$ für H, F, Cl oder Br stehen.

R in Formel I ist bevorzugt unsubstituiertes oder substituiertes $C_6$-$C_{10}$-Aryl, zum Beispiel Phenyl, 1- oder 2-Naphthyl. Bevorzugt stellt R unsubstituiertes oder substituiertes Phenyl dar.

Die Gruppe R kann mit einem oder mehreren, bevorzugt 1 bis 3 Resten substituiert sein. Wenn R mit Alkyl, Alkoxy oder Alkylthio substituiert ist, können diese linear oder verzweigt sein und bevorzugt 1 bis 6, besonders 1 bis 4 C-Atome enthalten. Beispiele sind Methyl, Ethyl, die Isomeren von Propyl, Butyl, Pentyl, Hexyl, Heptyl, Octyl, Nonyl, Decyl, und die entsprechenden Alkoxy- und Alkylthioreste. Bevorzugt sind Methyl, Ethyl, n- und i-Propyl, n-, i- und t-Butyl, Methoxy, Ethoxy, Methylthio und Ethylthio.

Wenn R mit Halogen substituiert ist, handelt es sich bevorzugt um -Br, -Cl und -F.

$R_5$ in der Bedeutung von Alkyl kann linear oder verzweigt sein. Weitere Beispiele zu den zuvorgenannten Alkylresten sind die Isomeren von Tridecyl, Tetradecyl, Pentadecyl, Hexadecyl, Heptadecyl und Octadecyl. Bevorzugt enthält $R_5$ als Alkyl 1 bis 12, besonders 1 bis 6 C-Atome. $R_5$ als Alkylphenyl ist bevorzugt $C_1$-$C_6$-, besonders $C_1$-$C_4$-Alkylphenyl, z. B. Dodecylphenyl, Octylphenyl, Hexylphenyl, n-, i- oder t-Butylphenyl, n- oder i-Propylphenyl, Ethylphenyl, Methylphenyl. $R_5$ als Alkylbenzyl ist bevorzugt $C_1$-$C_6$-, besonders $C_1$-$C_4$-Alkylbenzyl, z.B. Dodecylbenzyl, Octylbenzyl, Hexylbenzyl, n-, i- oder t-Butylphenyl, n- oder i-Propylbenzyl, Ethylbenzyl, Methylbenzyl. $R_5$ ist bevorzugt H oder $C_1$-$C_{18}$-Alkyl, besonders $C_1$-$C_{12}$-Alkyl.

In einer bevorzugten Ausführungsform ist R in Formel I unsubstituiert oder mit $C_1$-$C_4$-Alkyl, $C_1$-$C_4$-Alkoxy, $C_1$-$C_4$-Alkylthio, -F, -Cl, -Br oder $-COOR_5$ substituiert, wobei $R_5$ H oder $C_1$-$C_{18}$-Alkyl bedeutet.

Eine andere bevorzugte Ausführungsform sind solche Verbindungen der Formel I, worin in Formel I mindestens einer der Reste $R_1$ bis $R_4$ einen organischen Thio-, Sulfoxyl- oder Sulfonylrest darstellt, und die anderen Reste von $R_1$ bis $R_4$ für H stehen.

In einer weiteren bevorzugten Ausführungsform sind $R_1$ oder $R_4$, oder $R_1$ und $R_3$ oder $R_4$, oder $R_1$ und $R_2$ oder $R_1$ bis $R_4$ ein organischer Thio-, Sulfoxyl- oder Sulfonylrest. Der organische Thio-, Sulfoxyl- und Sulfonylrest enthält bevorzugt 1 bis 30, besonders 1 bis 20 und insbesondere 1 bis 12 C-Atome. $R_1$ bis $R_4$ stellen bevorzugt einen organischen Thiorest dar.

Bevorzugt sind Verbindungen der Formel I, worin der organische Thio-, Sulfoxyl- und Sulfonylrest den For-

meln $R_6S$-, $R_6SO$- oder $R_6SO_2$- entspricht, worin $R_6$ $C_1$-$C_{20}$-Alkyl, $C_3$-$C_8$-Cycloalkyl, $C_3$-$C_8$-Cycloalkylmethyl, $C_6$-$C_{10}$-Aryl oder $C_6$-$C_{10}$-Arylmethyl bedeutet, und $R_6$ unsubstituiert oder mit Halogen, -CN, $-CF_3$, $-COOR_5$, $C_1$-$C_{12}$-Alkyl, $C_1$-$C_{12}$-Alkoxy oder $C_1$-$C_{12}$-Alkylthio substituiert ist, und $R_5$ die zuvor angegebenen Bedeutungen hat. Bevorzugt ist der organische Rest ein Rest der Formel $R_6S$-.

$R_6$ kann unsubstituiert oder ein- oder mehrfach, besonders ein- bis dreifach substituiert sein. Wenn der Substituent Halogen ist, handelt es sich bevorzugt um -F, -Cl und -Br. Für $R_6$ gelten die zuvor angegebenen Bevorzugungen. Wenn der Substituent Alkyl, Alkoxy oder Alkylthio ist, so enthalten diese Reste bevorzugt 1 bis 6, besonders 1 bis 4 C-Atome. Beispiele für solche Reste sind zuvor aufgeführt worden.

In einer bevorzugten Ausführungsform ist $R_6$ unsubstituiert oder mit $C_1$-$C_4$-Alkyl, $C_1$-$C_4$-Alkoxy, -F, -Cl oder $-COOR_6$ substituiert, wobei $R_6$ H oder $C_1$-$C_{18}$-Alkyl bedeutet.

$R_6$ enthält als lineares oder verzweigtes Alkyl bevorzugt 1 bis 18 und besonders 1 bis 12 C-Atome. Beispiele sind zuvor angegeben worden. $R_6$ ist als Alkyl bevorzugt mit $-COOR_5$ substituiert und enthält dann im Alkyl bevorzugt 1 bis 4, besonders 1 oder 2 C-Atome; Beispiele sind $R_6OOC$-$CH_2S$- und $R_5OOC$-$CH_2CH_2S$-.

$R_6$ in der Bedeutung von Cycloalkyl enthält bevorzugt 4 bis 7, besonders 5 oder 6 Ring-C-Atome. Beispiele sind Cyclopropyl, Cyclobutyl, Cycloheptyl, Cyclooctyl und besonders Cyclopentyl und Cyclohexyl.

$R_6$ in der Bedeutung von Cycloalkylmethyl enthält bevorzugt 3 bis 7, besonders 5 oder 6 Ring-C-Atome, und ist besonders Cyclopentylmethyl und Cyclohexylmethyl.

$R_6$ als Aryl kann 1- oder 2-Naphthyl und besonders Phenyl sein.

$R_6$ als Arylmethyl kann Naphthylmethyl und besonders Benzyl sein.

In einer bevorzugten Ausführungsform stellt $R_6$ unsubstituiertes oder substituiertes $C_1$-$C_{12}$-Alkyl, Phenyl oder Benzyl dar.

Besonders bevorzugt sind Verbindungen der Formel I, worin $R_6$ $C_1$-$C_{12}$-Alkyl oder mit $-COOR_5$ substituiertes $C_1$-$C_4$-Alkyl bedeutet, oder Phenyl oder Benzyl darstellt, die unsubstituiert oder mit -F, -Cl, $C_1$-$C_4$-Alkyl, $C_1$-$C_4$-Alkoxy oder $-COOR_5$ substituiert sind, und $R_5$H oder $C_1$-$C_{18}$-Alkyl bedeutet.

Ein weiterer Gegenstand der Erfindung ist ein Verfahren zur Herstellung von Verbindungen der Formel I, bei dem man (a) eine Verbindung der Formel II

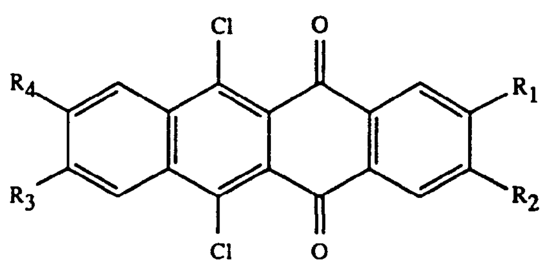

(II),

worin mindestens einer der Reste $R_1$, $R_2$, $R_3$ und $R_4$ einen organischen Thiorest bedeutet und die anderen Reste von $R_1$ bis $R_4$ für H, F, Cl oder BR stehen, in Gegenwart eines polaren aprotischen Lösungsmittels und bei erhöhter Temperatur mit einer Verbindung der Formel $RO^{\ominus}M^{\oplus}$ umsetzt, worin R die zuvor angegebenen Bedeutungen hat und M für ein Alkalimetall steht, und (b) die nach dem Verfahren (a) erhaltenen Verbindungen in an sich bekannter Weise zu Verbindungen der Formel I mit organischen Sulfoxyl- oder Sulfonylresten oxidiert.

Das erfindungsgemässe Verfahren wird bevorzugt bei Temperaturen von 50 bis 200°C, besonders 50 bis 150°C durchgeführt. Die Salze der Formel $RO^{\ominus}M^{\oplus}$ können als solche eingesetzt werden oder durch Umsetzung eines entsprechenden Phenols mit einer Alkalimetallbase oder Alkalimetallcarbonaten in situ im Reaktionsgemisch erzeugt werden. Die Salze können in äquimolaren Mengen oder im Ueberschuss, z.B. bis zu 40 Mol-% Ueberschuss eingesetzt werden.

Geeignete Lösungsmittel sind z.B. N-substituierte Carbonsäureamide und Lactame (z.B. Dimethylformamid oder N-Methylpyrrolidon), Sulfoxide und Sulfone (z.B. Dimethylsulfoxid, Tetramethylensulfon) oder Ether (z.B. n-Dipropylether, n-Dibutylether, Tetrahydrofuran oder Dioxan).

Die Oxidation der Verfahrensstufe (b) kann z. B. mit Alkalimetallperoxiden, organischen Peroxiden und bevorzugt mit $H_2O_2$ durchgeführt werden. Die Reaktion wird im allgemeinen in einem Lösungsmittel durchgeführt, wobei bevorzugt Eisessig als Lösungsmittel verwendet wird. Die Reaktionstemperatur kann von Raumtemperatur bis 150 °C, bevorzugt bis 100 °C betragen.

Die Isolierung und Reinigung der Verbindungen der Formel I erfolgt nach üblichen Methoden, z.B. durch Kristallisation und Umkristallisation, oder chromatographische Verfahren.

Die Verbindungen der Formel $RO^{\ominus}M^{\oplus}$ sind bekannt oder in bekannter Weise durch die Umsetzung entsprechender Phenole mit Alkalimetallbasen oder Alkalimetallcarbonaten erhältlich. Als Alkalimetallkationen

kommen besonders $Li^{\oplus}$, $Na^{\oplus}$ und $K^{\oplus}$ in Frage.

Ein weiterer Gegenstand der Erfindung sind Verbindungen der Formel II

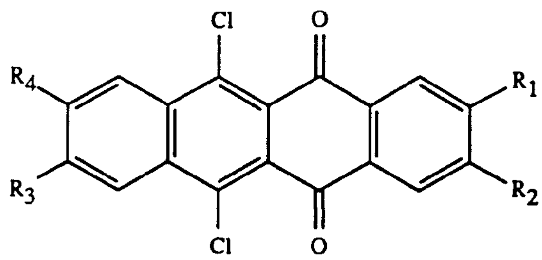

(II),

worin mindestens einer der Reste $R_1$ bis $R_4$ einen organischen Thiorest darstellt und die anderen für H, -F, -Cl oder -Br stehen. Für die Reste $R_1$ bis $R_4$ gelten die gleichen Bevorzugungen wie für die Verbindungen der Formel I.

Die Verbindungen der Formel II sind nach folgendem Verfahren erhältlich:

Die Umsetzung der bekannten Verbindungen der Formel III

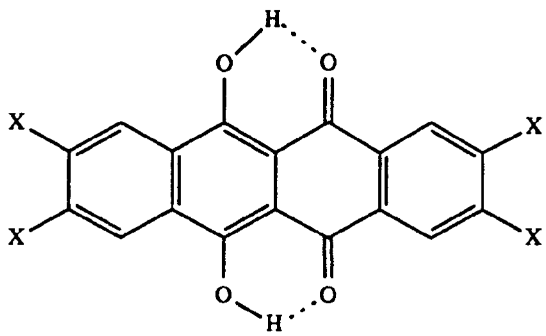

(III),

worin mindestens ein X Halogen, besonders F oder Cl und die anderen X Halogen oder H bedeuten, mit Organothiolen der Formel $R_1SH$, $R_2SH$, $R_3SH$ und/oder $R_4SH$ ergibt die Verbindung der Formel IV, die bei unsymmetrischer Substitution als Gemisch von Tautomeren der Formeln IV und IVa vorliegt:

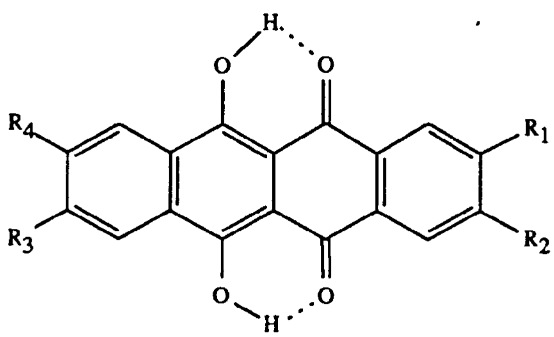

(IV)

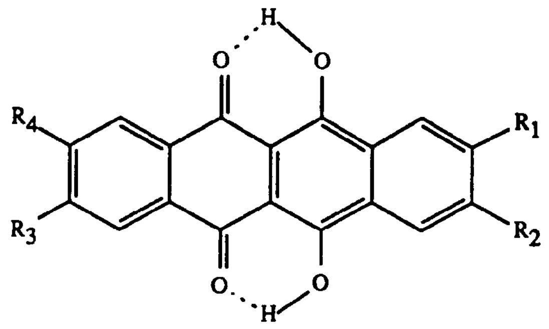

(IVa)

Diese Tautomerengemische können direkt zur Herstellung von Verbindungen der Formel II weiterverwendet werden oder vorher z.B. mit chromatographischen Methoden getrennt werden. Die nucleophile Substitution kann auch mit den entsprechenden Thiolatanionen durchgeführt werden, z.B. den Alkalimetallsalzen, besonders Li-, Na- und K-Salzen. Die Verbindungen der Formel III sind zum Beispiel durch die Umsetzung von entsprechend halogenierten bzw. nichthalogenierten Phthalsäureanhydriden mit entsprechend halogenierten bzw. nicht halogenierten 1,4-Dihydroxynaphthalin in Gegenwart von $B_2O_3$ bei erhöhten Temperaturen erhältlich.

Die Verbindungen der Formel IV bzw. IVa können mit üblichen Chlorierungsmitteln wie z.B. $POCl_3$ in die Verbindungen der Formel II übergeführt werden.

Die Verbindungen der Formel I mit $R_1$ bis $R_4$ gleich mindestens einem organischen Thiorest können auch hergestellt werden, indem man die Verbindungen der Formel III mit einem Chlorierungsmittel zunächst zu Verbindungen der Formel VI

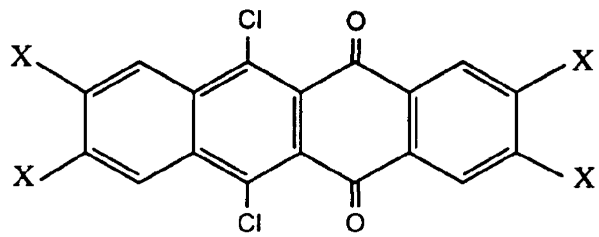

(VI)

chloriert, z. B. mit $POCl_3$ bei Temperaturen von 50 bis 200 °C und in Gegenwart eines Lösungsmittels, z. B. Dichlorbenzol, die Verbindungen der Formel VI in Gegenwart eines Lösungsmittels und eines Alkalimetallcarbonats bei Temperaturen von 50 bis 200 °C mit mindestens 3 Mol $R_1SH$ und gegebenenfalls mindestens 1 Mol $R_2SH$, $R_3SH$ und/oder $R_4SH$ pro Mol Verbindung der Formel VI zu Verbindungen der Formel VII

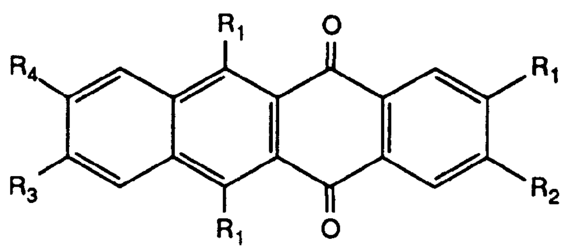

(VII)

umsetzt, worin $R_1$ einen organischen Thiorest und $R_2$ bis $R_4$ für einen organischen Thiorest, -H, -F, -Cl oder -Br stehen, und dann die Verbindungen der Formel VII unter oxidativen Bedingungen in Gegenwart eines Lösungsmittels und eines Alkalimetallcarbonats mit einem Phenol ROH zu Verbindungen der Formel I umsetzt. Geeignete Lösungsmittel sind zuvor genannt worden. Bevorzugte Alkalimetallcarbonate sind Natriumcarbonat und besonders Kaliumcarbonat. Oxidative Bedingungen bedeutet bevorzugt die Gegenwart von Luft. Bei diesem Verfahren werden überraschend nur die organischen Thiogruppen $R_1$ in den 5- und 11-Stellungen regioselektiv durch Phenolgruppen substituiert. Das Verfahren wird vorteilhaft zur Herstellung von Verbindungen der Formel I mit $R_1$ bis $R_4$ in der Bedeutung als organischer Thiorest verwendet.

Die Verbindungen der Formel I sind kristallin, thermisch stabil und gelb bis orange gefärbt. Sind in organischen Lösungsmitteln löslich. Sie sind wirksame Photoinitiatoren und Photosensibilisatoren für photopolymerisierbare Systeme, die ethylenisch ungesättigte Doppelbindungen enthalten. Ferner sind die Verbindungen der Formel I reversibel photochrom.

Bei Bestrahlung der erfindungsgemässen Verbindungen, gegebenenfalls in einem Substrat, mit Licht einer Wellenlänge von etwa 300 bis 450 nm wird eine ausgeprägte Farbänderung nach rot beobachtet. Die Lichtabsorption ist im Vergleich zu 6,11-Diphenoxynaphthacen-5,12-dion zu höherer Wellenlänge verschoben. Die Farbänderung beruht auf der photochemischen Umwandlung der erfindungsgemässen Parachinone in die entsprechenden Anachinone der Formel V. Die Umwandlungsgeschwindigkeit ist überraschend hoch und kann je nach Menge, Dicke der Probe und Strahlungsintensität unter 3 Sekunden liegen.

Ein weiterer Gegenstand der Erfindung sind die Anachinone der Formel V

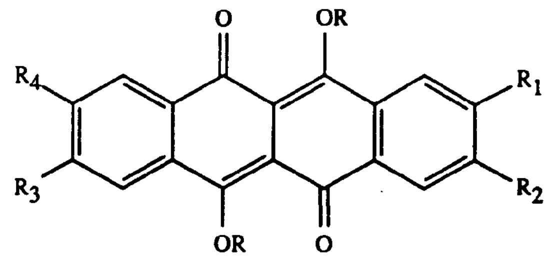

(V)

worin R, $R_1$, $R_2$, $R_3$ und $R_4$ die zuvor angegebenen Bedeutungen haben, einschliesslich der Bevorzugungen.

Die Verbindungen der Formel V können nach der Bestrahlung von Lösungen der Verbindungen der Formel I durch Entfemen des Lösungsmittels erhalten und gegebenenfalls nach üblichen Methoden gereinigt werden.

Die Farbänderung ist reversibel. Bei erneuter Bestrahlung mit Licht einer Wellenlänge von etwa 450 bis 550 nm erhält man wieder die ursprüngliche Farbe (Rückbildung der Parachinonstruktur). Besonders vorteilhaft ist, dass dieser Vorgang mehrfach wiederholt werden kann. Die Stabilität der photochemischen Hin- und Rückreaktion ist überraschend hoch und die Ermüdung selbst an Luft oder in Substraten entsprechend gering. So werden bei mehr als 200 Zyklen praktisch keine Veränderungen beobachtet. Als vorteilhaft ist auch anzusehen, dass die zur photochemischen Umwandlung benötigte Lichtabsorption im Bereich der Wellenlänge handelsüblicher Laser liegt.

Ein weiterer Gegenstand der Erfindung ist die Verwendung von Verbindungen der Formeln I oder V als reversible photochrome Systeme zur Kontrastbildung oder Lichtabsorption.

Die Verbindungen der Formel I können als Photoinitiatoren und besonders Photosensibilisatoren in photopolymerisierbaren Systemen verwendet werden, wobei sie gleichzeitig als Farbindikatoren wirken. So ist es möglich, belichtete Produkte (z.B. Schutzschichten, Druckplatten, Offsetdruckplatten, gedruckte Schaltungen, Lötstoppmasken) zu markieren und von unbelichteten Produkten zu unterscheiden, und ferner bei einer Produktekontrolle fehlerhaft belichtete Produkte vor oder nach der Entwicklung auszusondern.

Der erhebliche Vorteil bei der Verwendung als Farbindikatoren liegt in der Erhöhung der Sensibilisatorwirkung. Ueblicherweise als Farbumschlagsysteme eingesetzte Zusätze bewirken im allgemeinen eine Erniedrigung der Photosensibilität.

Die Verbindungen der Formeln I oder V können auch als solche, in Lösung oder in Polymeren eingearbeitet, als Photofarbindikatoren oder als Photoschaltelemente verwendet werden.

Die Verbindungen der Formel I können auch in organischen oder anorganischen Gläsern als photoschaltbare Farbfilter verwendet werden, z.B. in Gläsern für Sonnenbrillen, Kontaktlinsen, Fenstern und Spiegeln.

Ein weiterer Gegenstand der Erfindung ist eine strahlungsempfindliche Zusammensetzung, enthaltend

a) ein strahlungsempfindliches organisches Material, und

b) mindestens eine Verbindung der Formeln I, oder V oder Mischungen davon.

Die Verbindungen der Formel I und V oder Mischungen davon können in einer Menge von 0,001 bis 20 Gew.-%, besonders 0,001 bis 10 Gew.-% und insbesondere 0,01 bis 5 Gew.-% enthalten sein, bezogen auf die Komponente a).

Strahlungsempfindliche und damit auch photostrukturierbare Materialien sind bekannt. Es kann sich um Positiv- oder Negativsysteme handeln. Solche Materialien sind z.B. von G. E. Green et al. in J. Macromol. Sci.; Revs. Macromol. und Chem., C21(2), 187-273 (1981 bis 1982) und von G.A. Delzenne in Adv. Photochem., 11, S. 1-103 (1979) beschrieben worden.

Vorzugsweise handelt es sich bei dem strahlungempfindlichen organischen Material um a1) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide.

Photopolymerisierbare Substanzen sind z.B. Acryl- und besonders Methacrylsäureester von Polyolen, z.B. Ethylenglykol, Propandiol, Butandiol, Hexandiol, Di(hydroxymethyl)-cyclohexan, Polyoxyalkylendiole wie z.B. Di-, Tri- oder Tetraethylenglykol, Di- oder Tri- 1,2-propylenglykol, Trimethylolmethan, -ethan oder -propan und Pentaerythrit, die alleine, in Mischungen und in Abmischung mit Bindemitteln verwendet werden können.

Photodimerisierbare Substanzen sind z.B. Homo- und Copolymere, die Zimtsäuregruppen oder substituierte Maleinimidylverbindungen in Seitengruppen oder Chalkongruppen in der Polymerkette enthalten.

Bevorzugt sind solche Zusammensetzungen, worin Komponente a1) ein Homo- oder Copolymer von Acryl-, Methacryl- oder Maleinsäureestern ist, deren Estergruppen einen Rest der Formel

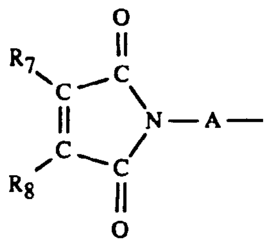

enthalten, worin A für unsubstituiertes oder mit Hydroxyl substituiertes lineares oder verzweigtes $C_2$-$C_{12}$-Alkylen, Cyclohexylen oder Phenylen steht, und $R_7$ und $R_8$ unabhängig voneinander Cl, Br, Phenyl oder $C_1$-$C_4$-Alkyl bedeuten, oder $R_7$ und $R_8$ zusammen Trimethylen, Tetramethylen oder

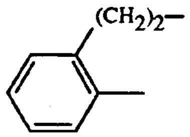

bedeuten. Solche Polymere sind z.B. in der US-A-4 193 927 beschrieben.

Die photopolymerisierbaren oder photodimerisierbaren Substanzen können weitere für die Verarbeitung oder Anwendung übliche Additive enthalten, sowie zusätzlich andere Photoinitiatoren oder Photosensibilisatoren.

Bei den kationisch härtbaren Systemen handelt es sich bevorzugt um Epoxidverbindungen mit mindestens 2 Epoxidgruppen im Molekül, denen ein Photoinitiator einverleibt ist. Geeignete Photoinitiatoren sind z.B. Cyclopentadienyl-Aren-Metallsalze, Cyclopentadienyl-metallcarbnoyl-salze und Oniumsalze, die z.B. in den zuvor erwähnten Publikationen beschrieben sind. Die härtbaren Systeme können für die Verarbeitung und Anwendung übliche Zusatzstoffe enthalten.

Photoempfindliche Polyimide sind z.B. in der DE-A-1 962 588, EP-A-0 132 221, EP-A-0 134 752, EP-A-0 162 017, EP-A-0 181 37 und EP-A-0 182 745 beschrieben.

Die erfindungsgemässe Zusammensetzung wird nach bekannten Methoden als Schicht auf Substrate aufgebracht und entweder durch flächige Bestrahlung eine Schutzschicht oder durch Bestrahlung unter einer Photomaske oder durch ortsaufgelöste Bestrahlung mittels eines geführten Laserstrahls oder durch holographische Verfahren und nachfolgende Entwicklung ein Reliefbild erzeugt.

Ein weiterer Gegenstand der Erfindung ist eine Zusammensetzung, enthaltend

a) ein farbloses organisches Lösungsmittel, ein Polymer oder ein organisches Glas oder ein Verbundglas, und

b) gelöst, eingemischt oder als Schicht auf mindestens einer Oberfläche eine Verbindung der Formel I oder V oder Mischungen davon. Die Komponente b) ist bevorzugt in einer Menge von 0,001 bis 20 Gew.-%, besonders 0,001 bis 10 Gew.-% und insbesondere 0,01 bis 5 Gew.-% enthalten, bezogen auf Komponente a). Organische Lösungen können zur Beschichtung von anderen Substanzen verwendet werden, z.B. festen Substraten wie zum Beispiel anorganischen Gläsern, die dann als photoschaltbare Substrate verwendet werden können. Die Verbindungen der Formel I oder V können auch auf Substrate aufsublimiert werden. Die beschichteten Substrate können mit einer Schutzschicht aus z.B. transparenten Polymeren versehen werden. Feste Substrate können auch mit Zusammensetzungen beschichtet werden, die ein Polymer und mindstens eine Verbindung der Formeln I oder V enthalten. Geeignete Lösungsmittel sind z.B. Kohlenwasserstoffe, halogenierte Kohlenwasserstoffe, Ketone, Carbonsäureester und Lactone, N-alkylierte Säureamide und Lactame, Alkanole und Ether.

Geeignete Polymere sind z.B. Duroplaste, Thermoplaste und strukturell vernetzte Polymere. Die Polymeren sind bevorzugt transparent. Solche Polymere und organische Gläser sind dem Fachmann geläufig. Die Einarbeitung der erfindungsgemässen Verbindungen erfolgt nach üblichen Methoden, z.B. mit Lösungsverfahren und Entfernen des Lösungsmittels, Kalandrieren oder Extrusion. Die erfindungsgemässen Verbindungen können den Substraten auch vor, während oder nach deren Herstellung einverleibt werden.

Ein weiterer Gegenstand der Erfindung ist ein Verfahren zur Herstellung von gefärbten Materialien unter Einwirkung von Licht, dadurch gekennzeichnet, dass man dem Material eine Verbindung der Formel I oder V einverleibt und darauf das Material mit Licht bestrahlt.

Ferner ist ein Gegenstand der Erfindung die Verwendung von Verbindungen der Formel I als Photosensibilisatoren und Farbindikatoren oder photoschaltbare Farbfilter bei Lichteinwirkung.

Ein weiterer Gegenstand der Erfindung ist die Verwendung der Verbindung der Formel I oder V zur reversiblen optischen Speicherung von Information, wobei die Information in einer die Verbindung enthaltenden, speicheraktiven Schicht mit Licht, bevorzugt Laserlicht, eingeschrieben wird. Die eingeschriebene Information kann mit bevorzugt Laserlicht, wieder gelöscht werden, so dass ein zyklisches Einschreiben und Löschen möglich ist.

Zur Herstellung einer speicheraktiven Schicht kann die Verbindung I oder V nach zuvor beschriebenen Verfahren in einer transparenten Matrix gelöst und in dünner Schicht auf ein ebenes Substrat aufgebracht werden. Die Dicke der speicheraktiven Schicht beträgt ca 0,1-100 µm, bevorzugt 0,3-3 µm.

Das Einschreiben der Information kann durch gerasterte, holographische oder photographische Belichtung der speicheraktiven Schicht mit spektralem, bevorzugt kohärentem (Laser-)Licht im Wellenlängenbereich 440-550 nm, bevorzugt 480-530 nm erfolgen.

Das Auslesen kann mit reduzierter Strahlungsleistung bei der Einschreibwellenlänge über die örtlich geänderte Transmission, Reflexion, Brechung oder Fluoreszenz der speicheraktiven Schicht erfolgen.

Das Löschen kann durch punktförmige oder flächige Belichtung der speicheraktiven, die Verbindungen der Formeln I und /oder V enthaltenden Schicht im Wellenbereich 300-450 nm, bevorzugt 300-400 nm erfolgen.

Ein Vorteil der erfindungsgemässen Verwendung ist, dass die zum Einschreiben, Auslesen und Löschen erforderlichen Wellenlängen im Bereich handelsüblicher Laser liegen (z.B. Argonionenlaser: 488/514 nm bzw. 351/363 nm; Neodym-YAG-Laser. 532 nm bzw. 355 nm; XeF-Excimerlaser: 351 nm; HeCd-Laser: 325 und 442 nm, bei Frequenzverdopplung und Verdreifachung).

Ein weiterer Vorteil ist der hohe erreichbare Kontrast der Absorption zwischen dem beschriebenen und dem gelöschten Zustand im Bereich 450-550 nm und die damit verbundene grosse Dynamik der speicheraktiven Schicht.

Ein anderer Vorteil ist, dass die Quantenausbeute beim Einschreiben relativ niedrig ist und damit die Gefahr eines Ueberschreibens beim Auslesen stark reduziert wird.

Vorteilhaft ist auch umgekehrt, dass die Quantenausbeute beim Löschvorgang relativ hoch ist und damit ein rasches grossflächiges Löschen ermöglicht wird.

Ein weiterer Vorteil ist, dass die Verbindung beim Auslesen fluoresziert und somit eine hochsensitive Detektion des Speicherzustands über die Fluoreszierung ermöglicht. Dass die zum Auslesen eingestrahlte Energie wesentlich über die Fluoreszierung und nicht thermisch dissipiert, wirkt zudem einer unerwünschten Erwärmung der speicheraktiven Schicht entgegen.

Ein weiterer Vorteil ist die hohe Photostabilität der Verbindung und die dadurch erreichbare hohe Zahl von Schreib-/Löschzyklen.

Schliesslich besteht als weiterer Vorteil die Möglichkeit einer zyklischen Datenauffrischung durch Zumischen eines geeigneten Quantums Licht der Löschwellenlänge während des Auslesens.

Die nachfolgenden Beispiele erläutern die Erfindung.

A) Herstellung von Ausgangsverbindungen

Beispiel A1: 2,3,8,9-Tetraphenylthio-6,11-dihydroxy-naphthacen-5,12-dion.
50 g (116,8 mMol) 2,3,8,9-Tetrachlor-6,11-dihydroxy-naphthacen-5,12-dion, 77,22 g (700,8 mMol) Thiophenol, 129,15 g (934 mMol) Kaliumcarbonat und 400 ml Dimethylsulfoxid (DMSO) werden während 1 Tag bei 100°C gerührt. Das Gemisch wird auf verdünnte wässrige HCl-Lösung ausgetragen und ausgerührt. Das rote Rohprodukt wird abfiltriert, mit Wasser gewaschen, bei 140°C im Vakuum getrocknet, dreimal mit Cyclohexan ausgekocht und wieder getrocknet. Ausbeute 80,11 g (95 %); Schmelzpunkt >260°C. MS: 722 ($M^+$; Basispeak).

Beispiel A2: 2,3,8,9-Tetraethylthio-6,11 -dihydroxy-naphthacen-5,12-dion.
Die Titelverbindung wird analog Beispiel A1 unter Verwendung von Ethylmerkaptan hergestellt. Ausbeute: 89

%; Smp.: 240°C (Zersetzung);MS: 530 ($M^+$; Basispeak).

Beispiel A3: 2,3,8,9-Tetra-n-dodecyl-6,11-dihydroxy-naphthacen-5,12-dion.

Die Titelverbindung wird analog Beispiel A1 unter Verwendung von Dodecylmerkaptan bei 120°C unter Zusatz von Dimethylformamid (DMF) hergestellt. Ausbeute: 87 %; Smp.: 66-76°C; MS: 1090 ($M^+$; Basispeak).

Beispiel A4: 2,3,8,9-Tetra(p-methylphenylthio)-6,11-dihydroxy-naphthacen-5,12-dion.

Die Titelverbindung wird analog Beispiel A1 unter Verwendung von p-Thiokresol bei 100°C hergestellt. Ausbeute: 83 %; Smp.: >265°C; MS: 778 ($M^+$; Basispeak).

Beispiel A5: 2,3,8,9-Tetra(p-chlorphenylthio)-6,11-dihydroxy-naphthacen-5,12-dion.

Die Titelverbindung wird analog Beispiel A1 unter Verwendung von p-Chlorthiophenol bei 120°C hergestellt. Ausbeute: 82 %; Smp.: >300°C; MS: 858/860/862/864 ($M^+$; Basispeak).

Beispiel A6: 2,3,8,9-Tetra(3-methoxyphenylthio)-6,11-dihydroxy-naphthacen-5,12-dion.

Die Titelverbindung wird analog Beispiel A1 unter Verwendung von m-Methoxythiophenol bei 100°C hergestellt. Ausbeute: 90 %; Smp.: 287-92°C; MS: 842 ($M^+$; Basispeak).

Beispiel A7 : 2-Phenylthio-6,11-dihydroxy-naphthacen-5,12-dion (Tautomerengemisch).

4,62 g (15 mMol) 2-Fluor-6,11-dihydroxy-naphthacen-5,12-dion (Tautomerengemisch), 2,2 g (20 mMol) Thiophenol, 11,06 g (80 mMol) Kaliumcarbonat und 50 ml Dimethylsulfoxid (DMSO) werden während 22 h bei 70°C gerührt. Das Gemisch wird auf 600 ml 0,5 M HCl-Lösung in $H_2O$ ausgetragen. Nach 15 Minuten unter Rühren wird der Niederschlag abfiltriert, dreimal mit Wasser und einmal mit Methanol gewaschen, bei 120°C im Vakuum getrocknet und aus Toluol umkristallisiert. Ausbeute 3,02 g (51 %), Smp. >260 °C; MS: 398 ($M^+$; Basispeak), 388, 295, 261, 233, 199, 176.

Analog zu Beispiel A7 werden unter Verwendung von 2-Chlor-6,11-dihydroxy-naphthacen-5,12 dion (Tautomerengemisch) und entsprechender substituierter Thiophenole bei 80 °C die Verbindungen A7a bis A7e (Tautomerengemische) erhalten.

A7a: 2- bzw. 9-(4'-Chlorphenylthio)-6,11-dihydroxy-naphthacen-5,12-dion.

Ausbeute 95 %, Smp.: 225-227 °C; Massenspektrum: 432 ($M^+$; Basispeak).

A7b: 2- bzw. 9-(4'-Fluorphenylthio)-6,11-dihydroxy-naphthacen-5,12-dion.

Ausbeute: 89 %, Smp.: 192-200 °C; Massenspektrum: 416 ($M^+$; Basispeak).

A7c: 2- bzw. 9-(4'-Methoxyphenylthio)-6,11-dihydroxy-naphthacen-5,12-dion.

Ausbeute: 84 %, Smp.: 170-175 °C; Massenspektrum: 428 ($M^+$: Basispeak)

A7d: 2- bzw. 9-(4'-Ethoxycarbonylphenylthio)-6,11-dihydroxy-naphthacen-5, 12-dion

Ausbeute: 95 %, Smp.: 140-150 °C; Massenspektrum: 470 ($M^+$: Basispeak).

A7e: 2- bzw. 9-(3'-Methoxyphenylthio)-6,11-dihydroxy-naphthacen-5,12-dion.

Ausbeute: 90 %, Smp.: 145-155 °C; Massenspektrum: 428 ($M^+$: Basispeak).

Beispiel A8: 2-n-Dodecylthio-5,11-dihydroxy-naphthacen-5,12-dion (Tautomerengemisch)

1,54 g (5 mMol) 2-Fluor-5,11-dihydroxy-naphthacen-5,12-dion (Tautomerengemisch), 5,53 g (40 mMol) $K_2CO_3$, 2,02 g (10 mMol) n-Dodecanthiol und 15 ml DMSO werden während 18 h bei 90°C gerührt. Das Gemisch wird auf verdünnte wässrige HCl-Lösung ausgetragen. Nach 10 Minuten Rühren wird das Produkt abfiltriert, dreimal mit Wasser und einmal mit Methanol gewaschen, bei 80°C getrocknet im Vakuum und aus Toluol umkristallisiert. Ausbeute 2,40 g (98 %), Smp. 146-148°C; MS: 398 ($M^+$; Basispeak), 388, 295, 261, 233, 199, 176.

Beispiel A9: 2,3,6,8,9, 11-Hexachlor-naphthacen-5,12-dion.

30 g (70 mmol) 2,3,8,9-Tetrachlor-6,11-dihydroxy-naphthacen-5,12-dion, 60 ml $POCl_3$ und 500 ml o-Dichlorbenzol werden 90 Stunden unter Rückfluss gerührt. Darauf wird der Ueberschuss des $POCl_3$ zusammen mit dem o-Chlorbenzol abdestilliert, bis das Reaktionsvolumen noch etwa 300 ml beträgt. Nach dem Abkühlen wird der Niederschlag abfiltriert, mehrmals mit Wasser und mit wässriger Sodalösung gewaschen, getrocknet, mit Cyclohexan verrührt, abfiltriert und dann getrocknet. Ausbeute: 28,6 g (88 %),SMP.: >260 °C.

B) Herstellung von erfindungsgemässen Verbindungen

Beispiel B1 : 2,3,8,9-Tetraethylthio-6,11-bis(3,5-dichlorphen-1-yloxy)-naphthacen-5,12-dion.

a) 0,30 g (0,57 mMol) Verbindung gemäss Beispiel A2 werden in 20 ml Phosphoroxychlorid während 8 Tagen am Rückfluss gerührt. Das Gemisch wird unter Kühlen auf Wasser ausgetragen und ausgerührt. Der Niederschlag wird abfiltriert, mit Wasser gewaschen und getrocknet. Nach der Chromatographie mit viel Methylenchlorid an Kieselgel werden 0,13 g (40 %) 2,3,8,9-Tetraethylthio-6,11-dichlor-naphthacen-5,12-dion erhalten, Smp. >260°C; MS: 566/568/570 ( Basispeaks, $M^+$).

b) 0,07 g (0,12 mMol dieser Verbindung, 0,05 g (0,31 mMol) 3,5-Dichlorphenol, 0,07 g (0,49 mMol) Kaliumcarbonat und 3 ml DMSO werden während 18 Stunden bei 70°C gerührt. Das Gemisch wird auf Wasser/Toluol ausgetragen, die organische Phase über Natriumsulfat getrocknet und eingedampft. Das Rohprodukt (0,08 g; 80 %), ist gelborange und weist im UV/VIS-Spektrum in Toluol gelöst folgende $\lambda_{max}$-Werte

auf: 439, 466 und 495 nm. Das Rohprodukt enthält wenig 2,3,8,9-Tetraethylthio-6,12-bis(3,5-dichlorphen-1-yloxy)-naphthacen-5,11-dion.

a) Analog dem Verfahren a) von Beispiel B1 wird ausgehend von Verbindung gemäss Beispiel A1 unter Zusatz von Dichlorbenzol als Lösungsmittel bei 160°C 2,3,8,9-Tetraphenylthio-6,11-dichlornaphthacen-5,12-dion erhalten; MS: 758/760/762 ( Basispeaks, $M^+$).

b) Analog dem Verfahren b) von Beispiel B1 wird durch Umsetzung dieser Verbindung mit Phenol die gelb-orange Titelverbindung erhalten. UV/VIS-Spektrum in Toluol: $\lambda_{max}$= 432 nm

Beispiel B3: 2-Phenylthio-6,11-diphenyloxy-naphthacen-5,12-dion (A) und 9-Phenylthio-6,11-diphenyloxy-naphthacen-5,12-dion (B).

a) 2,90 (7,28 mMol) Verbindung gemäss Beispiel A7 werden in 29 ml $POCl_3$ während 2 Tagen am Rückfluss gerührt. Das Gemisch wird unter starkem Rühren auf 300 ml Eiswasser ausgetragen und ausgerührt. Die Suspension wird mit Toluol extrahiert, die organischen Phasen werden filtriert, mit 2N NaOH-Lösung gewaschen, über Natriumsulfat getrocknet und eingedampft. Ausbeute: 2,73 g (86 %). Durch fraktionierte Kristallisation aus Toluol werden beide Isomere rein erhalten:

2-Phenylthio-6,11-dichlor-naphthacen-5,12-dion: 0,38 g; Smp. 201-3°C; MS: 434/436/438 ($m^+$).

9-Phenylthio-6,11-dichlor-naphthacen-5,12-dion: 0,13 g; Smp. 181-4°C; MS: 434/436/438 ($m^+$).

b) 2 g (4,59 mMol) des Rohproduktes (Isomerengemisch), 1,08 g ( 11,49 mMol) Phenol, 2,54 g (18,38 mMol) Kaliumcarbonat und 15 ml DMSO werden während 40 Minuten bei 60°C gerührt. Das Gemisch wird abgekühlt, mit THF/Toluol und verdünnter HCl-Lösung versetzt und ausgeschüttelt. Die organischen Phasen werden zweimal mit Wasser gewaschen, über Natriumsulfat getrocknet und eingedampft. Durch Flash-Chromatographie mit Methylenchlorid an Kieselgel werden die beiden Isomeren rein erhalten.

Schneller laufendes Produkt:

Titelverbindung A: 1,12 g Rohprodukt aus Toluol umkristallisiert: 0,78 g (31 %), Smp. 160-3°C; MS: 550 ($M^+$/Basispeak).

Langsamer laufendes Produkt:

Titelverbindung B: 0,77 g roh, aus Toluol umkristallisiert: 0,72 g (29 %), Smp. 150-3°C; MS (m/e): 550 ($M^+$) (Basispeak).

Beispiele B4-B8: Analog dem Verfahren b) von Beispiel B3 werden durch Umsetzung des Isomerengemisches von Beispiel B3a) mit dem entsprechenden Phenol die Isomerengemische der folgenden Verbindungen erhalten:

$R_1 = H$ $R_4 = SC_6H_5$

$R_1 = SC_6H_5$ $R_4 = H$

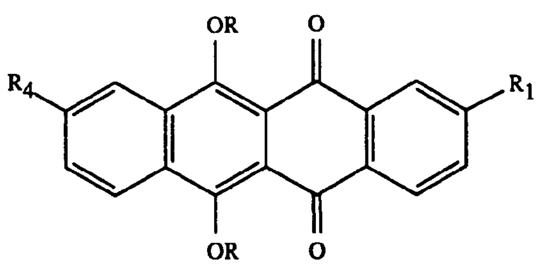

| R | Massenspektrum [M+] | Ausbeute [ % ] | Schmelzpunkt [ °C ] |
|---|---|---|---|
| 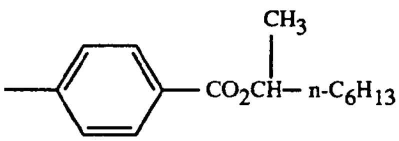 | 862 | 94 | Oel |
| 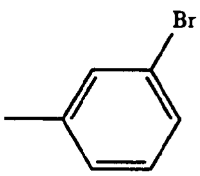 | 706 | 80 | 205 - 230°C |
| 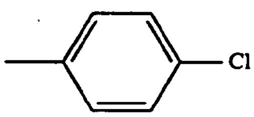 | 618 | 77 | 228 - 245°C |
| 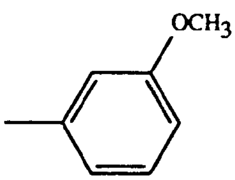 | 610 | 80 | 177 - 182°C |
| 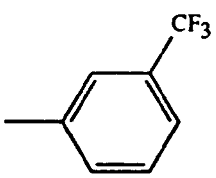 | 686 | 74 | 215 - 234°C |

Beispiel B9 : 2-n-Dodecylthio-6,11-diphenyloxy-naphthacen-5,12-dion (A) und 9-n-Dodecylthio-6,11-diphenyloxy-naphthacen-5,12-dion (B).

a) Analog zum Verfahren a) von Beispiel B3 werden unter Verwendung von n-Dodecylmerkaptan und dem Tautomerengemisch von Beispiel A8 nach der chromatographischen Trennung des Rohprodukts (Ausbeute 86 %) an Kieselgel mit Toluol erhalten: 2-n-Dodecylthio-6,11-dichlor-naphthacen-5,12-dion: 0,29 g; Smp. 120-125 °C; 9-n-Dodecylthio-6,11-dichlor-naphthacen-5,12-dion: 0,29 g; Smp. 113-114°C;

b) 0,20 g (0,38 mMol) 2-n-Dodecylthio-6, 11-dichlor-naphthacen-5,12-dion, 0,09 g (0,95 mMol) Phenol, 0,21 g ( 1,52 mMol) Kaliumcarbonat und 2 ml DMSO werden während 75 Minuten bei 60°C gerührt. Das Gemisch wird in Toluol aufgenommen und mit Toluol zweimal extrahiert. Die Toluolphasen werden zweimal mit Wasser gewaschen, über Natriumsulfat getrocknet und eingedampft. Aus Ether/Pentan umkristallisiert, werden 0, 13 g (54 %) der Titelverbindung A erhalten, Smp. 165-8°C; MS: 642 ($M^+$; Basispeak)

Analog wird aus 9-n-Dodecylthio-6,11-dichlor-naphthacen-5,12-dion die Titelverbindung B erhalten: Ausbeute nach Umkristallisation aus Ether/Pentan: 0,13 g (54 %), Smp. 143-5°C; MS: 642 ($M^+$; Basispeak).

Beispiele B10-B14: Substituierte Phenylthioreste enthaltende Naphthacendione.

Die Verbindungen A7a bis A7e werden analog dem Verfahren B3a chloriert und analog dem Verfahren B3b mit Phenol zu den entsprechenden Isomerengemischen umgesetzt:

Beispiel B10: 2- bzw. 9-(4'-Chlorphenylthio)-6,11-diphenyloxy-naphthacen-5,12-dion.

a) 2- bzw. 9-(4'-Chlorphenylthio)-6, 11-dichlor-naphthacen-5,12-dion:Reaktionszeit: 7 Tage, Ausbeute: 64 %, Massenspektrum: 468 ($M^+$: Basispeak).

b) Titelverbindung: Ausbeute: 70 %, Smp.: >250 °C, Massenspektrum: 584 ($M^+$: Basispeak).

Beispiel B11: 2- bzw. 9-(4'-Fluorphenylthio)-naphthacen-6,11-diphenyloxy-naphthacen-5,12-dion.

a) 2- bzw. 9-(4'-Fluorphenylthio)-6,11-dichlor-naphthacen-5,12-dion: Reaktionszeit: 8 Tage, Ausbeute: 68 %, Massenspektrum: 452 ($M^+$: Basispeak).

b) Titelverbindung: Ausbeute: 61 %, Smp.: >250 °C, Massenspektrum: 568 ($M^+$: Basispeak).

Beispiel B12: 2-bzw. 9-(4'-Methoxyphenylthio)-6,11-phenyloxy-naphthacen-5,12-dion

a) 2- bzw. 9-(4'-Methoxyphenylthio)-6,11-dichlor-naphthacen-5,12-dion: Reaktionszeit: 4 Tage, Ausbeute: 85 %, Massenspektrum: 464 ($M^+$: Basispeak).

b) Titelverbindung: Ausbeute: 66 %, Smp.: 200-206 °C, Massenspektrum: 580 ($M^+$: Basispeak).

Beispiel B13: 2- bzw. 9-(4'-Ethoxycarbonylphenylthio)-6,11-diphenyloxy-naphthacen-5,12-dion.

a)2- bzw. 9-(4'-Ethoxycarbonylphenylthio)-5,11-dichlor-naphthacen-5,12-dion: Reaktonszeit: 7 Tage, Ausbeute: 25 %, Massenspektrum: 506 ($M^+$: Basispeak).

b) Titelverbindung: Ausbeute: 83 %, Smp.: 190-210 °C, Massenspektrum: 622 ($M^+$: Basispeak).

Beispiel B14: 2- bzw. 9-(3'-Methoxyphenylthio)-6, 11-diphenyloxy-naphthacen-5,12-dion

a) 2- bzw. 9-(3'-Methoxyphenylthio)-6,11-dichlor-naphthacen-5,12-dion: Reaktionszeit: 6 Tage, Ausbeute: 85 %, Massenspektrum: 464 ($M^+$: Basispeak).

b) Titelverbindung: Ausbeute: 75 %, Smp.: 160-170 °C, Massenspektrum: 580 ($M^+$: Basispeak).

Beispiel B15: Isomerengemisch von 2,3- und 8,9-Di(phenylthio)-6,1 1-diphenyloxy-naphthacen-5,12-dion.

a) 2,3- und 8,9-Diphenylthio-6,11-dihydroxy-naphthacen-5,12-dion 5g (13,9 mmol) 2,3- und 8,9-Dichlor-6,11-dihydroxy-naphthacen-5,12-dion, 3,37 g (30,6 mmol) Thiophenol, 6,81 g (49,3 mmol) $K_2CO_3$ und 50 ml Dimethylsulfoxid werden 3 Stunden bei 65 °C gerührt. Das Gemisch wird in verdünnte Salzsäure ausgetragen, die roten Kristalle abfiltriert, mit Wasser gewaschen und im Vakuum bei 120 °C getrocknet. Nach dem Umkristallisieren aus Toluol erhält man 4,5 g Produkt, Smp.: >270 °C.

b) 2,3- und 8,9-Diphenylthio-6,11-dichlor-naphthacen-5,12-dion 2,5 g des nach Stufe a) erhaltenen Isomerengemischs und 45 ml $POCl_3$ werden 4 Tage unter Rückfluss gerührt. Das Gemisch wird auf Wasser ausgetragen, die Kristalle abfiltriert, mehrmals mit Wasser gewaschen und dann in Toluol gelöst. Die abgetrennte organische Phase mit verdünnter Natronlauge, dann mit Wasser gewaschen, danach über Natriumsulfat getrocknet und eingedampft.Nach Verrühren mit Toluol unter Zusatz von Aluminiumoxid, Filtrieren und Eindampfen erhält man 1,81 g gelbe Kristalle, Smp.: 240-243 °C.

c) 1,7 g (3,13 mmol) des nach Stufe b) erhaltenen Isomerengemischs 0,74 g (7,82 mmol) Phenol, 1,3 g (9,4 mmol) Kaliumcarbonat und 30 ml Dimethylsulfoxid werden 1 Stunde bei 85 °C gerührt. Dann wird das Reaktionsgemisch in verdünnte Salzsäure ausgetragen, der kristalline Niederschlag abfiltriert und in Tetrahydrofuran/Toluol (1:1) aufgenommen. Die organische Phase wird mit Wasser gewaschen, über Natriumsulfat getrocknet und dann eingedampft. Nach Umkristallisation aus Toluol erhält man 1,14 g (55 %) der Titelverbindung als gelbe Kristalle, Smp.: >270 °C, Massenspektrum: 658 ($M^+$, Basispeak). Beim Belichten einer Toluollösung beobachtet man eine reversible Farbänderung von gelb-orange nach rot.

Beispiel B16: 2,8- bzw. 2,9-Diphenylthio-6,11-diphenyloxy-nahthacen-5,12-dion.

a) 2,6,8,11- bzw. 2,6,9, 11-Tetrachlor-naphthacen-5,12-dion.

20 g (55,8 mmol) 2,8- bzw. 2,9-Dichlor-6, 11-dihydroxy-naphthacen-5,12-dion (Tautomerengemisch), 100 ml $POCl_3$ und 400 ml o-Dichlorbenzol werden 5 Tage am Rückfluss erhitzt. Dann engt man auf etwa das halbe Volumen ein und versetzt mit Methylenchlorid. Die organische Phase wird abgetrennt, mit Wasser und gesättigter Kochsalzlösung gewaschen, über Natriumsulfat getrocknet und dann eingedampft. Der Rückstand wird in Methylenchlorid zweimal an Kieselgel chromatographiert und zweimal aus Xylol umkristallisiert. Man erhält 11,2 g Produkt (51 %).

b) 2,6,8,11 - bzw. 2,6,9,11,-Tetraphenylthio-naphthacen-5,12-dion

2 g des gemäss Stufe a) erhaltenen Isomerengemischs (5,05 mmol), 5,58 g (40,4 mmol) Kaliumcarbonat, 3,34 g (30,3 mmol) Thiophenol und 14 ml Dimethylsulfoxid werden 1 Tag bei 60 °C und 4 Stunden bei 120 °C gerührt. Dann giesst man auf verdünnte Salzsäure und extrahiert mit Toluol. Die organische Phase wird mit Wasser gewaschen, mit Natriumsulfat getrocknet und eingedampft. Das Rohprodukt wird in Toluol an Kieselgel chromatographiert. Man erhält 1,38 g (40 %) der Verbindung als rotes Oel.

c) 0,3 g der nach Stufe b) erhaltenen Verbindung (0,434 mmol), 0,14 g (1,52 mmol) Phenol, 0,24 g ( 1,74 mmol) Kaliumcarbonat und 10 ml Dimethylsulfoxid werden unter Einleiten von Luft bei 100 °C während 6

Stunden gerührt. Nach dem Abkühlen nimmt man in Toluol auf, wäscht nacheinander mit verdünnter Salzsäure, 1N Natronlauge und dann mit Wasser, trocnet über Natriumsulfat und dampft ein. Das Rohprodukt wird unter Lichtausschluss in Toluol na Kieselgel chromatographiert. Man erhält 0,12 g (43 %) der Titelverbindung als oranges Oel, Massenspektrum: 658 ($M^+$: Basispeak). Beim Bestrahlen einer Toluollösung wird eine reversible Farbänderung von gelb-orange nach rot beobachtet.

Beispiel B17: 2,3,8,9,-Tetraphenylthio-6,11-diphenyloxy-naphthacen-5, 12-dion

a) 2,3,6,8,9,11-Hexaphenylthio-naphthacen-5,12-dion.

10 g (21,5 mmol) Verbindung A9, 23,7 g (215 mmol) Thiophenol, 32,69 g (237 mmol) Kaliumcarbonat und 200 ml Dimethylsulfoxid werden 18 Stunden bei 80 °C gerührt. Nach dem Abkühlen wird in 2N Salzsäure ausgetragen, der Niederschlag abfiltriert und mit Wasser gewaschen. Der kristalline Niederschlag wird mehrmals mit heissem Cyclohexan extrahiert und dann getrocknet. Man erhält 16.8 g (86 %) Produkt als rote Kristalle, Smp.: >260 °C, Massenspektrum: 690 ($M^+$: Basispeak).

b) 8 g der gemäss Stufe a) hergestellten Verbindung (8,82 mmol), 4,14 g (44,1 mmol) Phenol, 7,31 g (52,9 mmol) Kaliumcarbonat und 150 ml Dimethylsulfoxid werden unter Einleiten von Luft 9 Stunden bei 100 °C gerührt und dann abgekühlt. Man giesst danach in 2N Salzsäure, filtriert den Niederschlag ab, wäscht mehrmals mit Wasser und trocknet den Niederschlag. Man kristallisiert aus Toluol um und 5,33 g (69 %) der Titelverbindung, Smp.: >270 °C; Massenspektrum: 874 ($M^+$); 782 und 690 (je $-C_6H_5$). In Toluollösung beobachtet man einen reversiblen Farbumschlag von gelb nach rot.

Beispiel B18: 2-Phenylsulfonyl-6,11-diphenyloxy-naphthacen-5,12-dion.

4 g (7,26 mmol) Verbindung B3 (Isomerengemisch) werden mit 5 ml 30 %-iger wässrigem $H_2O_2$ und 70 ml Eisessig während 8 Stunden bei 80 °C gerührt. Das Gemisch wird in Wasser/Tetrahydrofuran/Toluol aufgenommen und die organische Phase abgetrennt, mit wässriger Natriumbisulfit-Lösung und zweimal mit Wasser gewaschen, über Natriumsulfat getrocknet und eingedampft. Man kristallisiert aus Toluol um und erhält 2,08 g (49 %) der Titelverbindung, Smp.: 260-270 °C; Massenspektrum: 582 ($M^+$: Basispeak). Bei Belichtung einer Toluollösung beobachtet man einen reversiblen Farbumschlag von gelb nach orange.

C) Anwendungsbeispiele

Beispiel C1: Die Verbindungen der Beispiele B1 bis B3 sowie B9 werden in Lösung mit einer Xenonlampe (120 W) bestrahlt, wobei die entsprechenden Anachinone gebildet werden; z.B.

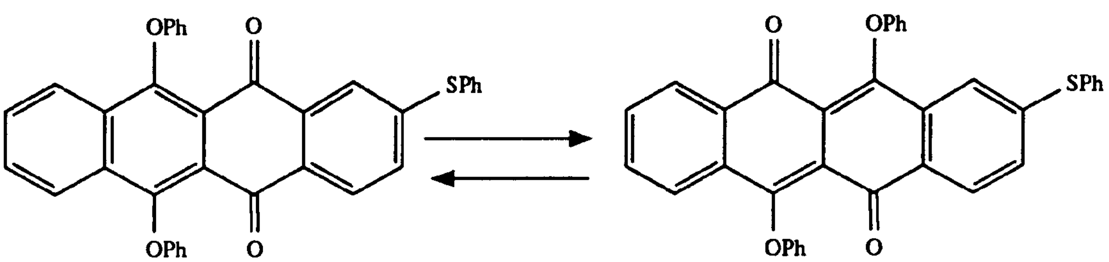

(I) (V)

Die Anachinone können durch Abdampfen des Lösungsmittels isoliert werden. Weitere Angaben sind in der nachfolgenden Tabelle enthalten.

| Verbindung von Beispiel | Lösungsmittel | Farbänderung | UV/VIS-Spektrum $\lambda_{max}$ (nm) | |
|---|---|---|---|---|
| | | | I | V |
| B 1 | Toluol | gelb-orange nach rot | 439, 469, 495 | 498, 552 |
| B 2 | Toluol | gelb-orange nach rot | 432 | 493, 528 |
| Verbindung A von B 3 | Acetonitril | gelb nach rot | 408 | 467, 503 |
| Verbindung B von B 3 | Acetonitril | gelb nach rot | 393 | 467, 503 |
| Verbindung A von B 9 | Acetonitril | gelb nach rot | 411 | 469, 502 |
| Verbindung B von B 9 | Acetonitril | gelb nach rot | 395 | 469, 502 |

Beispiel C2: Filme mit erfindungsgemässen Verbindungen.
100 mg Polystyrol und 3,05 mg Verbindung A (Film I) bzw. Verbindung B (Film II) von Beispiel B3 werden in 8,3 g Toluol gelöst. Je 1/5 der Lösungen wird auf beheizte Glasplatten von je 60, 70, 80, 90 und 100°C gegossen und das Toluol abgedampft. Die Filme sind gelb gefärbt und transparent.

Der Film I wird mit UV-Licht (334 nm, Laserstrahl, aufgeweitet auf 4,5 mm) bis zur Sättigung belichtet (Rotfärbung) und in den Strahlengang eines Diodenarray-Spektrometers gestellt. Während der Belichtung mit sichtbarem Laserlicht (488 nm, 125 mW/ $cm^2$) werden periodisch Transmissionsspektren aufgenommen. Die Photoreaktion wird aufgrund der spektralen Aenderung bei 470 nm verfolgt.
Die Konversionsrate bei dieser Belichtung ergibt eine Zeitkonstante der Umwandlung von 2.6 s.
Für die Rückreaktion genügt eine Laser-Intensität von 25 $mW/cm^2$ bei 325 nm für eine gleiche Geschwindigkeit.

Der Film I wird bei 325 nm mit 125 $mJ/cm^2$ belichtet, danach bei 488 nm mit 625 $mJ/cm^2$. Jeweils nach 10 Zyklen wird ein Spektrum aufgenommen und mit demjenigen vor dem ersten Zyklus verglichen. Nach 150 alternierenden Belichtungen ist keine permanente Veränderung der Probe messbar. Die zur Photoreaktion notwendige Energiedichte bleibt ebenfalls gleich.

Beispiel C3: Belichtungszyklen mit einem Polystyrolfilm.
100 mg Polystyrol und 3 mg Verbindung B3 werden in 8,3 g Toluol gelöst und auf eine 70 °C warme Glasplatte

gegossen. Nach dem Abdampfen des Lösungsmittels erhält man einen gelb gefärbten und transparenten Film mit einer Schichtdicke von 10 µm.

Der Film wird auf einer Quarzglasplatte im Probenraum eines Spektralphotometers montiert und mit einer 300 W Xenonlampe über Glasfasern und einen UV-Filter (Schott UG11) bestrahlt. Die integrale Bestrahlungsstärke beträgt 0,5 $mW/cm^2$. In Abständen von jeweils etwa 60 s wird die Bestrahlung unterbrochen und das Absorptionsspektrum gemessen. Das Spektrum der Probe ändert sich von gelb (optische Dichte 1 bei 300 nm, 0,4 bei 400 nm und null oberhalb 450 nm) nach rot, verursacht durch eine breite Absorptionsbande im Bereich von 400 bis 550 nm (maximale optische Dichte 0,65 bei 480 nm). Die Zeitkonstante der Umwandlung beträgt 250 s.

Für die Rückreaktion wird der UV-Filter durch ein gelbes Kantenfilter (Schott GG475) mit Durchlass oberhalb etwa 450 nm ausgetauscht. Die integrale Bestrahlungsstärke im Bereich 450 bis 550 nm beträgt 3 $mW/cm^2$. Durch die Bestrahlung verschwindet die langwellige Absorptionsbande bei 400 bis 550 nm bis auf eine optische Dichte von maximal 0, 1. Die Zeitkonstante der Rückreaktion beträgt 200 s.

Bei weiteren Belichtungszyklen bleiben die Grenzwerte der optischen Dichte konstant (bei 480 nm: etwa 0,1/0,65).

Beispiel C4: Punktweise Beschriftung eines Polystyrolfilms

Der Film gemäss Beispiel C3 wird zwischen 2 Quarzglasplatten auf einen xy-Tisch montiert und mit UV-Licht (Excimerlaser 308 nm, etwa 0,2 $J/cm^2$ pro Puls) bis zur Sättigung belichtet (Rotfärbung). Ueber einen elektrooptischen Schalter, eine Monomode-Faser und ein Mikroskopobjektiv wird punktweise Laserstrahlung der Wellenlänge 488 nm ($Ar^+$-Ionenlaser) auf den Film fokussiert. Je nach Bestrahlungsstärke und Pulsdauer beträgt die Transmission der eingeschriebenen Punkte bei 488 nm nach der Belichtung 25 % bis 85 %. Die erforderliche Besrahlungsleistung für 50 % Transmissionsänderung (25% auf 75%) beträgt 0,5 $W/cm^2$. Während des Einschreibens zeigen die bestrahlten Punkte orange Fluoreszens (500-700 nm). Zum Auslesen der Transmission eines Punktes genügt eine gegenüber dem Einschreiben wesentlich reduzierte Energie, welche die Transmission praktisch nicht verändert. Durch erneute UV-Bestrahlung des Films (etwa 0,2 $J/cm^2$ bei 308 nm) werden die Punkte nahezu vollständig gelöscht.

Beispiel C5: Aufzeichnung eines Hologramms.

Der Film gemäss Beispiel C3 wird zwischen zwei Quarzglasplatten in der Filmebene eines holographischen Aufzeichnungsgeräts montiert und mit UV-Licht bis zur Sättigung belichtet (Rotfärbung). Mit einem aufgeweiteten, raumgefilterten Strahl eines Argonlasers (488 nm) wird mittels eines Regenbogen-Masterhologramms ein Objekt senkrecht auf die Filmebene projiziert. Ein Teil des aufgeweiteten Strahls dient als Referenzwelle (Einfallswinkel etwa 30°). Die Bestrahlungsstärke in der Filmebene beträgt etwa 5 $mW/cm^2$, aufgeteilt auf die Referenz- und Objektwelle im Verhältnis 4: 1. Nach einer Belichtungszeit von 60 s wird die Bestrahlungsstärke wesentlich reduziert und die Objektwelle ausgeblendet. In der Filmebene erscheint mit gutem Kontrast das in der Folie aufgezeichnete Hologramm. Das Hologramm ist ebenso klar sichtbar, wenn zum Auslesen das Weisslicht einer Spotlampe statt Laserlicht verwendet wird, wobei nur die kurzwelligen (blauen) Regenbogenfarben auftreten. Nach längerer Bestrahlung des Films unter der Referenzwelle nehmen Helligkeit und Kontrast des Hologramms ab.

Beispiel C6: Holographische Aufzeichnung.

Der Film gemäss Beispiel C3 wird zwischen zwei Quarzglasplatten in der Filmebene eines holographischen Aufzeichnungsgeräts montiert. Aus einem aufgeweiteten, raumgefilterten Argonlaserstrahl (488 nm, $\phi$ etwa 0,5 cm) werden zwei ebene Wellen (O und R) von je 2,5 $mW/cm^2$ Bestrahlungsstärke gebildet und unter einem Winkel von 3° in der Filmebene zur Koinzidenz gebracht. Aus einer 300 W Xenonlampe wird über einen UV-Filter (Schott UG11) und eine Quarzfaser ein mit O und R koinzidierendes UV-Lichtbündel (0,5 $mW/cm^2$) auf die Filmebene gerichtet. Hinter der Filmebene wird in Richtung der ersten Beugungsordnung von R, bzw. der zweiten von O, ein Detektor zur Messung des Beugungswirkungsgrades (BWG) befestigt.

Der Film wird durch zehnminütige Bestrahlung mit UV-Licht in die rote Form übergeführt. Nach Einschalten von O und R steigt der BWG innerhalb von 60 s monoton von 0 auf etwa 0,1 % (einschreiben). Nach Unterbrechen von O steigt der BWG sprunghaft auf etwa 0,12 % (Wegfall der destruktiv interferierenden 2. Beugungsordnung von O) und nimmt dann näherungsweise exponentiell mit einer Zeitkonstante von etwa 60 s ab (überschreiben). Durch erneute UV-Bestrahlung wird wieder gelöscht. Nach 10 Zyklen ist keine Verminderung des BWG feststellbar.

Bei gleichzeitigem Einschreiben und Löschen (holographischer Kurzzeitspeicher) wird ein stationärer BWG von etwa 0,05 % erreicht.

## Patentansprüche

1. Verbindungen der Formel I oder Mischungen solcher Verbindungen,

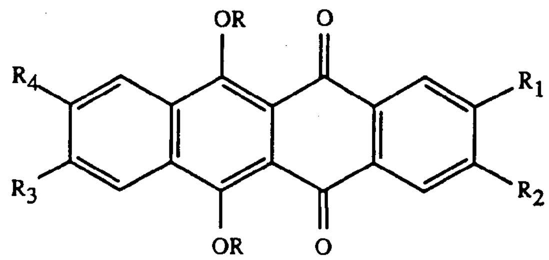

(I),

worin
R unsubstituiertes oder mit $C_1$-$C_{12}$-Alkyl, $C_1$-$C_{12}$-Alkoxy, $C_1$-$C_{12}$-Alkylthio, Phenyl, Benzyl, -CN, -$CF_3$, Halogen oder -$COOR_5$ substituiertes $C_6$-$C_{14}$-Aryl bedeutet und $R_5$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht, und
mindestens einer der Reste $R_1$ bis $R_4$ einen organischen Thio-, Sulfoxyl oder Sulfonylrest darstellt und die anderen Reste von $R_1$ bis $R_4$ für H, F, Cl oder Br stehen.

2. Verbindungen gemäss Anspruch 1, worin R in Formel I unsubstituiertes oder substituiertes $C_6$-$C_{10}$-Aryl ist.

3. Verbindungen gemäss Anspruch 2, worin R unsubstituiertes oder substituiertes Phenyl, 1- oder 2-Naphthyl bedeutet.

4. Verbindungen gemäss Anspruch 1, worin R in Formel I unsubstituiert oder mit $C_1$-$C_4$-Alkyl, $C_1$-$C_4$-Alkoxy, $C_1$-$C_4$-Alkylthio, -F, -Cl, -Br oder -$COOR_5$ substituiert ist, wobei $R_5$ H oder $C_1$-$C_{18}$-Alkyl bedeutet.

5. Verbindungen gemäss Anspruch 1, worin in Formel I mindestens einer der Reste $R_1$ bis $R_4$ einen organischen Thio-, Sufoxyl- oder Sulfonylrest darstellt, und die anderen Reste von $R_1$ bis $R_4$ für H stehen.

6. Verbindungen gemäss Anspruch 1, worin $R_1$ oder $R_4$, oder $R_1$ und $R_3$ oder $R_4$, oder $R_1$ und $R_2$ oder $R_1$ bis $R_4$ einen organischen Thio-, Sulfoxyl- oder Sulfonylrest darstellen.

7. Verbindungen gemäss Anspruch 1, worin der organische Thio-, Sulfoxyl und Sulfonylrest den Formeln $R_6S$-, $R_6SO$- und $R_6SO_2$- entspricht, worin $R_5$ $C_1$-$C_{20}$-Alkyl, $C_3$-$C_8$-Cycloalkyl, $C_3$-$C_8$-Cycloalkylmethyl, $C_6$-$C_{10}$-Aryl oder $C_6$-$C_{10}$-Arylmethyl bedeutet, und $R_6$ unsubstituiert oder mit Halogen, -CN, -$CF_3$, -$COOR_5$, $C_1$-$C_{12}$-Alkyl, $C_1$-$C_{12}$-Alkoxy oder $C_1$-$C_{12}$-Alkylthio substituiert ist, und $R_5$ die in Anspruch 1 angegebenen Bedeutungen hat.

8. Verbindungen gemäss Anspruch 7, worin $R_6$ unsubstituiert oder mit $C_1$-$C_4$-Alkyl, $C_1$-$C_4$-Alkoxy, -F, -Cl oder -$COOR_5$ substituiert ist, wobei $R_5$ H oder $C_1$-$C_{18}$-Alkyl bedeutet.

9. Verbindungen gemäss Anspruch 7, worin $R_5$ unsubstituiertes oder substituiertes $C_1$-$C_{12}$-Alkyl, Phenyl oder Benzyl ist.

10. Verbindungen gemäss Anspruch 7, worin $R_5$ $C_1$-$C_{12}$-Alkyl oder mit -$COOR_5$ substituiertes $C_1$-$C_4$-Alkyl bedeutet, oder Phenyl oder Benzyl darstellt, die unsubstituiert oder mit -F, -Cl, $C_1$-$C_4$-Alkyl oder $C_1$-$C_4$-Alkoxy oder -$COOR_5$ substituiert sind, und $R_5$ H oder $C_1$-$C_{18}$-Alkyl bedeutet.

11. Verfahren zur Herstellung von Verbindungen der Formel I gemäss Anspruch 1, bei dem man (a) eine Verbindung der Formel II

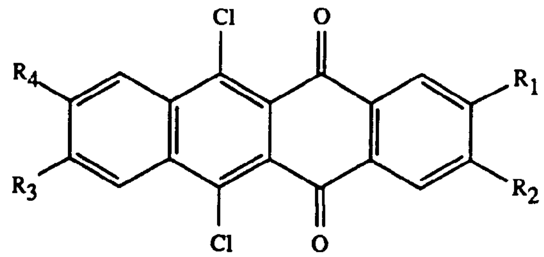

(II),

worin mindestens einer der Reste $R_1$, $R_2$, $R_3$ und $R_4$ einen organischen Thiorest bedeutet und die anderen Reste von $R_1$ bis $R_4$ für H, F, Cl oder Br stehen, in Gegenwart eines polaren aprotischen Lösungsmittels und bei erhöhter Temperatur mit einer Verbindung der Formel $RO^{\ominus}M^{\oplus}$ umsetzt, worin R die in Anspruch 1 angegebenen Bedeutungen hat und M für ein Alkalimetall steht, und (b) die nach dem Verfahren (a) erhaltenen Verbindungen zu Verbindungen der Formel I mit organischen Sulfoxyl- oder Sulfonylresten oxidiert.

**12.** Verbindungen der Formel II

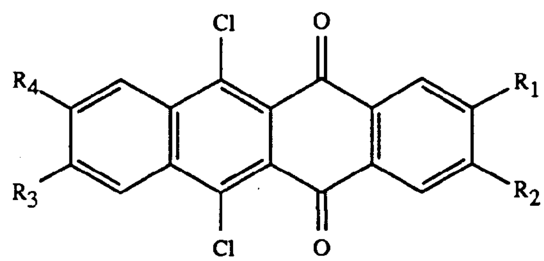

(II),

worin mindestens einer der Reste $R_1$ bis $R_4$ einen organischen Thiorest darstellt und die anderen für H, -F, -Cl oder -Br stehen.

**13.** Verbindungen gemäss Anspruch 12, worin in Formel II mindestens einer der Reste $R_1$ bis $R_4$ einen organischen Thiorest darstellen, und die anderen für H stehen.

**14.** Verbindungen gemäss Anspruch 12, worin $R_1$ oder $R_4$ oder $R_1$ bis $R_4$ einen organischen Thiorest darstellen.

**15.** Verbindungen gemäss Anspruch 12, worin der organische Thiorest der Formel $R_6$S-entspricht, worin $R_6$ $C_1$-$C_{20}$-Alkyl, $C_3$-$C_8$-Cycloalkyl, $C_3$-$C_8$-Cycloalkylmethyl, $C_6$-$C_{10}$-Aryl oder $C_6$-$C_{10}$-Arylmethyl bedeutet, und $R_6$ unsubstituiert oder mit Halogen, -CN, $-CF_3$, $-COOR_6$, $C_1$-$C_{12}$-Alkyl, $C_1$-$C_{12}$-Alkoxy oder $C_1$-$C_{12}$-Alkylthio substituiert ist, und $R_5$ die in Anspruch 1 angegebenen Bedeutungen hat.

**16.** Verbindungen gemäss Anspruch 15, worin $R_6$ unsubstituiert oder mit $C_1$-$C_4$-Alkyl, $C_1$-$C_4$-Alkoxy, -F, -Cl oder $-COOR_6$ substituiert ist, wobei $R_5$ H oder $C_1$-$C_{18}$-Alkyl bedeutet.

**17.** Verbindungen gemäss Anspruch 15, worin $R_6$ unsubstituiertes oder substituiertes $C_1$-$C_{12}$-Alkyl, Phenyl oder Benzyl ist.

**18.** Verbindungen gemäss Anspruch 15, worin $R_6$ $C_1$-$C_{12}$-Alkyl oder mit $-COOR_6$ substituiert substituiertes $C_1$-$C_4$-Alkyl bedeutet, oder Phenyl oder Benzyl darstellt, die unsubstituiert oder mit -F, -Cl, $C_1$-$C_4$-Alkyl oder $C_1$-$C_4$-Alkoxy substituiert sind, und $R_6$ H oder $C_1$-$C_{18}$-Alkyl bedeutet.

**19.** Verbindungen der Formel V

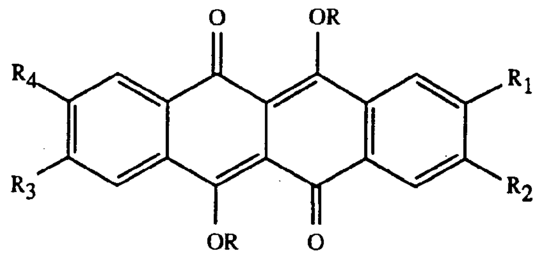

(V),

worin R, $R_1$, $R_2$, $R_3$ und $R_4$ die in Anspruch 1 angegebenen Bedeutungen haben.

20. Verbindungen der Formel I gemäss Anspruch 1, worin mindestens einer der Reste $R_1$ bis $R_4$ für einen organischen Thiorest steht.

21. Verbindungen der Formel I gemäss Anspruch 1, worin R für Phenyl steht und $R_1$, oder $R_1$ und $R_2$, oder $R_1$ und $R_3$, oder $R_1$ und $R_4$, oder $R_1$ bis $R_4$ für Phenylthio stehen und die anderen Reste von $R_2$ bis $R_4$ jeweils H bedeuten.

22. Zusammensetzung, enthaltend
a) ein farbloses organisches Lösungsmittel, ein Polymer oder ein organisches Glas oder ein Verbundglas, und
b) gelöst, eingemischt oder als Schicht auf mindestens einer Oberfläche eine Verbindung der Formel I oder V oder Mischungen davon.

23. Verwendung von Verbindungen der Formeln I oder V oder Mischungen davon als reversible photochrome Systeme zur Kontrastbildung oder Lichtabsorption.

24. Verwendung von Verbindungen der Formeln I oder V oder zur reversiblen optischen Speicherung von Informationen.

## Claims

1. A compound of formula I, or a mixture of such compounds,

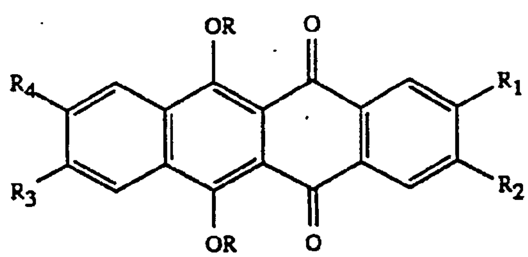

(I),

wherein
R is unsubstituted $C_6$-$C_{14}$aryl or $C_6$-$C_{14}$aryl which is substituted by $C_1$-$C_{12}$alkyl, $C_1$-$C_{12}$alkoxy, $C_{1}$-$_{12}$alkylthio, phenyl, benzyl, -CN, -$CF_3$, halogen or -$COOR_5$, and $R_5$ is H, $C_1$-$C_{18}$alkyl, cyclohexyl, cyclopentyl, phenyl, $C_1$-$C_{12}$alkylphenyl, benzyl or $C_1$-$C_{12}$alkylbenzyl, and at least one of the radicals $R_1$ to $R_4$ is an organic thio, sulfoxyl or sulfonyl radical, and the other radicals $R_1$ to $R_4$ are H, F, Cl or Br.

2. A compound according to claim 1, wherein R in formula I is unsubstituted or substituted $C_6$-$C_{10}$aryl.

3. A compound according to claim 2, wherein R is unsubstituted or substituted phenyl, or 1- or 2-naphthyl.

4. A compound according to claim 1, wherein R in formula I is unsubstituted or substituted by $C_1$-$C_4$alkyl, $C_1$-$C_4$alkoxy, $C_1$-$C_4$alkylthio, -F, -Cl, -Br or -$COOR_5$, and $R_5$ is H or $C_1$-$C_{18}$alkyl.

5. A compound according to claim 1, wherein in formula I at least one of the radicals $R_1$ to $R_4$ is an organic thio, sulfoxyl or sulfonyl radical and the other radicals $R_1$ to $R_4$ are H.

6. A compound according to claim 1, wherein $R_1$ or $R_4$, or $R_1$ and $R_3$ or $R_4$, or $R_1$ and $R_2$ or $R_1$ to $R_4$ are an organic thio, sulfoxyl or sulfonyl radical.

7. A compound according to claim 1, wherein the organic thio, sulfoxyl or sulfonyl radical has the formula $R_6S$-, $R_6SO$- or $R6SO_2$-, where $R_6$ is $C_1$-$C_{20}$alkyl, $C_3$-$C_8$cycloalkyl, $C_3$-$C_8$cycloalkylmethyl, $C_6$-$C_{10}$arylmethyl, and $R_6$ is unsubstituted or substituted by halogen, -CN, -$CF_3$, -$COOR_5$, $C_1$-$C_{12}$alkyl, $C_1$-$C_{12}$alkoxy or $C_1$-$C_{12}$alkylthio, and $R_5$ is as defined in claim 1.

8. A compound according to claim 7, wherein R6 is unsubstituted or substituted by $C_1$-$C_4$alkyl, $C_1$-$C_4$alkoxy, -F, -Cl or -$COOR_5$, and $R_5$ is H or $C_1$-$C_{18}$alkyl.

9. A compound according to claim 7, wherein R6 is unsubstituted or substituted $C_1$-$C_{12}$alkyl, phenyl or benzyl.

10. A compound according to claim 7, wherein $R_6$ is $C_1$-$C_{12}$alkyl or $C_1$-$C_4$alkyl which is substituted by -$COOR_5$, or is phenyl or benzyl, each unsubstituted or substituted by -F, -Cl, $C_1$-$C_4$alkyl, $C_1$-$C_4$alkoxy or -$COOR_5$, and $R_5$ is H or $C_1$-$C_{18}$alkyl.

11. A process for the preparation of a compound of formula I according to claim 1, which comprises (a) reacting a compound of formula II

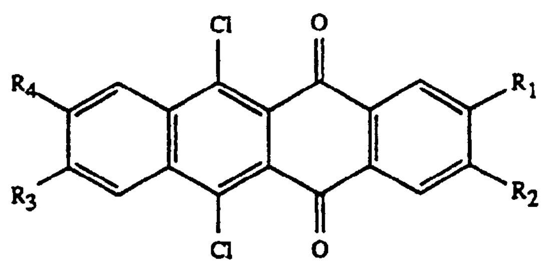 (II),

wherein at least one of the radicals $R_1$, $R_2$, $R_3$ and $R_4$ is an organic thio radical and the other radicals $R_1$ to $R_4$ are H, F, Cl or Br, in the presence of a polar aprotic solvent and at elevated temperature, with a compound of formula $RO^{\ominus}M^{\oplus}$, where R is as defined in claim 1 and M is an alkali metal, and (b) oxidising the compound obtained by process (a) to a compound of formula I containing organic sulfoxyl or sulfonyl radicals.

12. A compound of formula II

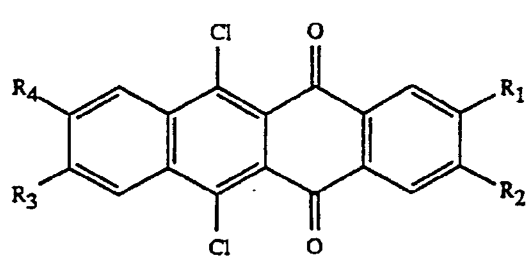 (II),

wherein at least one of the radicals $R_1$ to $R_4$ is an organic thio radical, and the others are H, -F, -Cl or -Br.

13. A compound according to claim 12, wherein in formula II at least one of $R_1$ to $R_4$ is an organic thio radical, and the others are H.

14. A compound according to claim 12, wherein $R_1$ or $R_4$ or $R_1$ to $R_4$ are an organic thio radical.

**15.** A compound according to claim 12, wherein the organic thio radical has the formula $R_6S$-, wherein $R_6$ is $C_1$-$C_{20}$alkyl, $C_3$-$C_8$cycloalkyl, $C_3$-$C_8$cycloalkylmethyl, $C_6$-$C_{10}$aryl or $C_6$-$C_{10}$arylmethyl, and $R_6$ is unsubstituted or substituted by halogen, -CN, $-CF_3$, $-COOR_5$, $C_1$-$C_{12}$alkyl, $C_1$-$C_{12}$alkoxy or $C_1$-$C_{12}$alkoxy or $C_1$-$C_{12}$alkylthio, and $R_6$ is as defined in claim 1.

**16.** A compound according to claim 15, wherein $R_6$ is unsubstituted or substituted by $C_1$-$C_4$alkyl, $C_1$-$C_4$alkoxy, -F, -Cl or $-COOR_6$, and $R_5$ is H or $C_1$ $C_{18}$alkyl.

**17.** A compound according to claim 15, wherein $R_6$ is unsubstituted or substituted $C_1$-$C_{12}$alkyl, phenyl or benzyl.

**18.** A compound according to claim 15, wherein $R_6$ is $C_1$-$C_{12}$alkyl or $C_1$-$C_4$alkyl which is substituted by $-COOR_5$, or is phenyl or benzyl, each unsubstituted or substituted by -F, -Cl, $C_1$-$C_4$alkyl or $C_1$-$C_4$alkoxy, and $R_6$ is H or $C_1$-$C_{18}$alkyl.

**19.** A compound of formula V

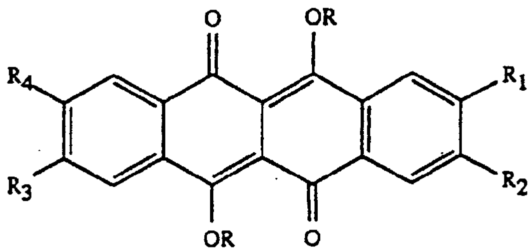

(V),

wherein R, $R_1$, $R_2$, $R_3$ and $R_4$ are as defined in claim 1.

**20.** A compound of formula I according to claim 1, wherein at least one of the radicals $R_1$ to $R_4$ is an organic thio radical.

**21.** A compound of formula I according to claim 1, wherein R is phenyl and $R_1$, or $R_1$ and $R_2$, or $R_1$ and $R_3$, or $R_1$ and $R_4$, or $R_1$ to $R_4$ are phenylthio and the other radicals $R_2$ to $R_4$ are each H.

**22.** A composition comprising
a) a colourless organic solvent, a polymer or an organic glass or a compound glass, and
b) dissolved, admixed or present as a layer on at least one surface, a compound of formula I or V or a mixture thereof.

**23.** Use of a compound of formula I or V or of a mixture thereof as a reversible photochromic system for contrast formation or light absorption.

**24.** Use of a compound of formula I or V for the reversible optical storage of information.

**Revendications**

**1.** Composés de formule I ou mélanges de ces composés

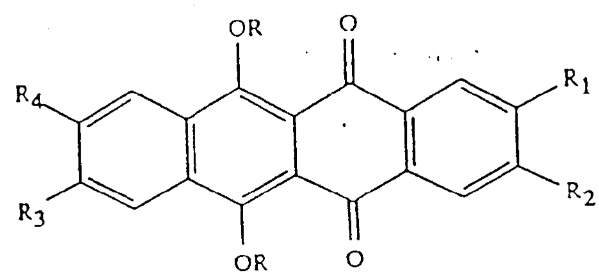

(I),

dans laquelle
R est un radical aryle en $C_6$-$C_{14}$, non substitué ou substitué par des radicaux alkyle en $C_1$-$C_{12}$, alcoxy en $C_1$ à $C_{12}$, alkylthio en $C_1$ à $C_{12}$, phényle, benzyle, -CN, $-CF_3$, halogéno, $-COOR_5$, et $R_5$ est H ou un radical alkyle en $C_1$ à $C_{18}$, cyclohexyle, cyclopentyle, phényle, (alkyle en $C_1$ à $C_{12}$) phényle, benzyle ou (alkyle en $C_1$ à $C_{12}$) benzyle et au moins un des radicaux $R_1$ à $R_4$ représente un radical thio, sulfoxyle ou sulfonyle organique, les autres radicaux de $R_1$ à $R_4$ représentant H, F, Cl ou Br.

2. Composés selon la revendication 1, dans lesquels R, dans la formule I, est un radical aryle en $C_6$ à $C_{10}$, éventuellement substitué.

3. Composés selon la revendication 2, dans lesquels R est un radical phényle, 1- ou 2-napthyle éventuellement substitués.

4. Composés selon la revendication 1, dans lesquels R, dans la formule I est non substitué ou substitué par des radicaux alkyle en $C_1$-$C_4$, alcoxy en $C_1$-$C_4$, alkylthio en $C_1$-$C_4$, -F, -Cl, -Br ou $-COOR_5$, $R_5$ étant H ou radical en $C_1$-$C_{18}$.

5. Composés selon la revendication 1, dans lesquels, dans la formule I, au moins l'un des radicaux $R_1$ à $R_4$ est un radical thio, sulfoxyle ou sulfonyle organique et les autres radicaux $R_1$ à $R_4$ sont des hydrogènes.

6. Composés selon la revendication 1, dans lesquels $R_1$ ou $R_4$, ou $R_1$ et $R_3$ ou $R_4$, ou $R_1$ et $R_2$ ou $R_1$ à $R_4$ représentent des radicaux thio, sulfoxyle ou sulfonyle, organiques.

7. Composés selon la revendication 1 dans lesquels les radicaux organiques thio, sulfoxyle et sulfonyle correspondent aux formules $R_6S$-, $R_5SO$- ou $R_5SO_2$-, où $R_5$ est un radical alkyle en $C_1$ à $C_{20}$, cycloalkyle en $C_3$ à $C_8$, (cycloalkyle en $C_3$ à $C_8$) méthyle, aryle en $C_6$ à $C_{10}$, ou (aryle en $C_6$ à $C_{10}$) méthyle et $R_5$ est non substitué, ou est substitué par des halogènes ou des radicaux -CN, $-CF_3$, $-COOR_5$, alkyle en $C_1$ à $C_{12}$, alcoxy en $C_1$ à $C_{12}$ ou alklythio en $C_1$ à $C_{12}$ et $R_5$ a les significations données dans la revendication 1.

8. Composés selon la revendication 7, dans lesquels $R_6$ est non substitué ou substitué par des radicaux alkyle en $C_1$ à $C_4$, alcoxy en $C_1$ à $C_4$, -F, -Cl ou $-COOR_5$, $R_5$ étant H ou un radical alkyle en $C_1$ à $C_{18}$.

9. Composés selon la revendication 7, dans lesquels $R_6$ est un radical alkyle en $C_1$ à $C_{12}$, phényle ou benzyle éventuellement substitués.

10. Composés selon la revendication 7, dans lesquels $R_5$ est un radical alkyle en $C_1$ à $C_{12}$ ou alkyle en $C_1$ à $C_4$ substitués par $-COOR_5$, ou encore un radical phényle ou benzyle, qui sont non substitués ou substitués par des radicaux -F, -Cl, alkyle en $C_1$ à $C_4$ ou alcoxy en $C_1$ à $C_4$ ou $-COOR_5$, $R_5$ est H ou un radical alkyle en $C_1$ à $C_{18}$.

11. Procédé pour préparer des composés de formule I selon la revendication 1, dans lequel (a) on fait réagir un composé de formule II

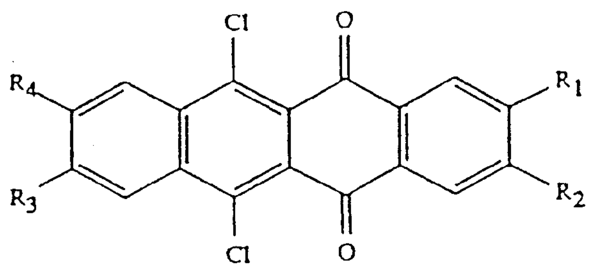

(II),

dans laquelle au moins l'un des radicaux $R_1$, $R_2$, $R_3$ et $R_4$ est un radical thio organique et les autres radicaux de $R_1$ à $R_4$ représentent H, F, Cl ou Br, en présence d'un solvant aprotique polaire et à haute température, avec un composé de formule $RO^-M^+$, où R a les significations données ci-dessus, et M est un métal alcalin, et (b) on oxyde les composés obtenus par le procédé (a), d'une manière connue en soi, pour obtenir des composés de formule I comportant des radicaux sulfoxyle ou sulfonyle organiques.

12. Composés de formule II

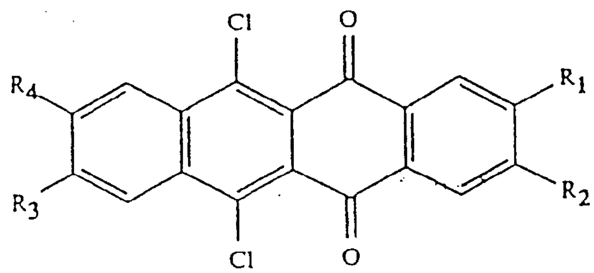

(II),

dans lesquels au moins l'un des radicaux $R_1$ à $R_4$ est un radical thio organique et les autres sont des radicaux h, -F, -Cl ou -Br.

13. Composés selon la revendication 12, dans lesquels, dans la formule II, au moins l'un des radicaux $R_1$ à $R_4$ est un radical thio organique et les autres sont des hydrogènes.

14. Composés selon la revendication 12, dans lesquels $R_1$ ou $R_4$ ou $R_1$ à $R_4$ sont des radicaux thio organiques.

15. Composés selon la revendication 12, dans lesquels les radicaux organiques thio, a la formule $R_6S$-, $R_6SO$- ou $R_6SO_2$-, où $R_6$ est un radical alkyle en $C_1$ à $C_{20}$, cycloalkyle en $C_3$ à $C_8$, (cycloalkyle en $C_3$ à $C_8$) méthyle, aryle en $C_6$ à $C_{10}$, ou (aryle en $C_6$ à $C_{10}$) méthyle et $R_6$ est non substitué, ou est substitué par des halogènes ou des radicaux -CN, $-CF_3$, $-COOR_6$, alkyle en $C_1$ à $C_{12}$, alcoxy en $C_1$ à $C_{12}$ ou alklythio en $C_1$ à $C_{12}$ et $R_6$ a les significations données dans la revendication 1.

16. Composés selon la revendication 15, dans lesquels $R_6$ est non substitué ou substitué par des radicaux alkyle en $C_1$ à $C_4$, alcoxy en $C_1$ à $C_4$, -F, -Cl ou $-COOR_5$, $R_5$ étant H ou un radical alkyle en $C_1$ à $C_{18}$.

17. Composés selon la revendication 15, dans lesquels $R_6$ est un radical alkyle en $C_1$ à $C_{12}$, phényle ou benzyle éventuellement substitués.

18. Composés selon la revendication 15, dans lesquels $R_6$ est un radical alkyle en $C_1$ à $C_{12}$ ou alkyle en $C_1$ à $C_4$ substitués par $-COOR_6$, ou encore un radical phényle ou benzyle, qui sont non substitués ou substitués par des radicaux -F, -Cl, alkyle en $C_1$ à $C_4$ ou alcoxy en $C_1$ à $C_4$ ou $-COOR_6$, $R_6$ est H ou un radical alkyle en $C_1$ à $C_{18}$.

19. Composés de formule V

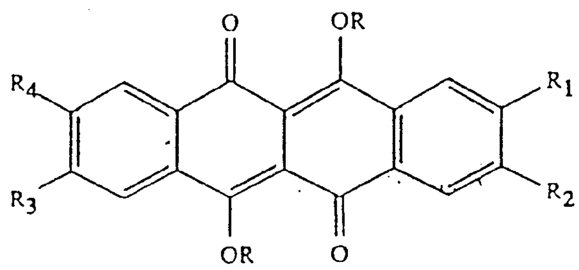

(V),

dans lesquels $R_1$, $R_2$, $R_3$ et $R_4$ ont les significations données dans la revendication 1.

**20.** Composés de formule I selon la revendication 1, dans lesquels au moins l'un des radicaux $R_1$ à $R_4$ est un radical thio organique.

**21.** Composés de formule I selon la revendication 1, dans lesquels R est le radical phényle, et $R_1$ ou $R_1$ et $R_2$ ou $R_1$ et $R_3$, ou $R_1$ et $R_4$, ou $R_1$ à $R_4$ sont des radicaux phénylthio, les autres radicaux de $R_2$ à $R_4$ étant chacun un hydrogène.

**22.** Composition contenant:

a) un solvant organique incolore, un polymère et un verre organique ou un verre feuilleté, et

b) en solution, en mélange ou sous forme d'une couche sur au moins une surface, un composé de formule I ou V, ou leurs mélanges.

**23.** Utilisation de composés de formules I ou V, ou de leurs mélanges en tant que systèmes photochromiques réversibles pour la formation du contraste ou la formation de la lumière.

**24.** Utilisation de composés de formules I ou V, ou de leurs mélanges, pour la mémorisation optique réversible d'informations.